# EUROPEAN PATENT SPECIFICATION

(11) **EP 4 261 220 B1**
(45) Date of publication and mention of the grant of the patent: **14.05.2025**
(21) Application number: 23159824.4
(22) Date of filing: 03.03.2023
(51) Int. Cl.: C07K 14/47, C12N 9/36, A61K 39/00, G01N 15/10

(54) **ANTIBODY COMBINATION FOR SUBSTITUTING SIDE SCATTER SIGNAL IN MASS CYTOMETRY HEMATOLOGIC TUMOR IMMUNOPHENOTYPING AND USE THEREOF**
ANTIKÖRPERKOMBINATION ZUR SUBSTITUTION EINES SEITENSTREUSIGNALS IN DER HÄMATOLOGISCHEN TUMORIMMUNOPHÄNOTYPISIERUNG IN DER MASSENZYTOMETRIE UND VERWENDUNG DAVON
COMBINAISON D'ANTICORPS PERMETTANT DE REMPLACER UN SIGNAL DE DIFFUSION LATÉRALE EN CYTOMÉTRIE DE MASSE, IMMUNOPHÉNOTYPAGE TUMORAL HÉMATOLOGIQUE ET SON UTILISATION

(30) Priority: 11.04.2022 CN 202210375754
(43) Date of publication of application: 18.10.2023
(73) Proprietor: Zhejiang Puluoting Health Technology Co., Ltd., Hangzhou, Zhejiang (CN)
(72) Inventor: LIN, cheng, Hangzhou City, Zhejiang Province, 310023 (CN); QIU, Chensheng, Hangzhou City, Zhejiang Province, 310023 (CN)
(74) Representative: Sun, Yiming

(56) References cited:
- ALAN D. STERN ET AL: "Cell size assays for mass cytometry", CYTOMETRY A, vol. 91, no. 1, 1 January 2017 (2017-01-01), Hoboken, USA, pages 14 - 24, XP055618595, ISSN: 1552-4922, DOI: 10.1002/cyto.a.23000
- LACOMBE F ET AL: "Flow cytometry CD45 gating for immunophenotyping of acute myeloid leukemia", LEUKEMIA, NATURE PUBLISHING GROUP UK, LONDON, vol. 11, no. 11, 1 November 1997 (1997-11-01), pages 1878 - 1886, XP037782176, ISSN: 0887-6924, DOI: 10.1038/SJ.LEU.2400847

## Description

### TECHNICAL FIELD

The present disclosure relates to the technical field of mass cytometry, in particular to an antibody combination for substituting a side scatter signal in mass cytometry hematologic tumor immunophenotyping and use thereof.

### BACKGROUND

To select a therapeutic regimen correctly, the precondition is an accurate classification of hematologic tumors. Currently, the mode that is commonly used in the world is cell morphology, immunology, cytogenetics and molecular biology classification, i.e., MICM classification. Among them, multi-parameter flow cytometry of immunology classification plays an important role, which improves the identification accuracy of specific disease types based on the immune signature of patient tumor cells.

As for the multi-parameter flow cytometry, CD molecules, such as stem/progenitor cell antigens, bone marrow cell line associated antigens, red blood cells, B cells, T cells, NK cells, megakaryocytes and other associated antigens, on the surface of bone marrow cells are detected by fluorescent antibodies. Common antibody combinations are usually three- or four-color schemes, using three or four fluoresceins to label antibodies separately. Flow cytometry is usually carried out for hematologic tumor classification by gating. That is, cell subsets are distinguished deeply step by step. For example, in T cells, the T cells are distinguished using CD3, and then CD3+CD4+T cells and CD3+CD8+T cells are distinguished using CD4 and CD8. The side scatter (SSC) signal of flow cytometers also plays an important role. At present, for the analysis of flow cytometry detection results, the first-stage gating strategy is to use CD45 and SSC as horizontal and vertical coordinates respectively to distinguish CD45 negative nucleated red blood cell subsets, CD45dimSSC-low primitive and juvenile cell subsets, CD45dimSSC-high mature granulocyte subsets, CD45 + SSC-low lymphocyte subsets, and CD45 + SSC-intermediate monocyte subsets, and further analyze each subset by different antibodies.

For completing hematologic tumor classification, it usually needs to detect 30 or more CD molecule antibodies and some other classification antibodies. Since commonly used flow cytometers in clinic is of 4-6 color, i.e., it can detect 4-6 proteins on a cell at a time. To complete the detection of 30 or more antibodies, a tube of bone marrow needs to be divided into 8-10 tubes of samples for staining and analysis respectively (some antibodies require repeated detection to determine cell subsets), resulting in large sample sizes. The process is cumbersome and it is not possible to carry out simultaneous analysis of 30 or more protein parameters for a single cell. The multi-parameter flow cytometry is also interfered by background fluorescence of samples. The emission wavelengths of different fluoresceins are overlapped. Therefore, even light filters are used, compensation regulation is still required.

Mass cytometry is a new multi-parameter flow cytometry that uses metal-labeled antibodies with extremely low abundance in organisms such as rare earth metals, and uses time-of-flight mass spectrometry to accurately detect the metal content in each cell. Due to the detection characteristics of mass spectrometers, there is almost no interference between different metal signals and no compensation regulation is required. It is possible to simultaneously detect 43 antibodies (including CD molecules) on a single cell with single-tube detection, which has a methodological advantage for cell classification of complex cell types, overcomes the defects of the current 4-6 color traditional flow cytometry, and has the potential to be used as a hematologic tumor immunodetection platform.

For detection of hematologic tumors, the traditional flow cytometry uses CD45 and SSC for gating, which can quickly distinguish nucleated red cell subsets, primitive and juvenile cell subsets, monocyte subsets, lymphocyte subsets, mature granulocyte subsets, and the like. Since mass cytometry uses mass spectrometry methodology, in which cells are completely ionized, SSC of traditional flow cytometry is not included in the detection process. When applied to hematologic tumors, the first-stage gating similar to flow cytometry, namely CD45 and SSC combined gating, cannot be carried out, and the major cell subsets cannot be distinguished. Therefore, the clinical application and scientific research of mass cytometry in the hematologic tumor field are limited. Thus, it is necessary to develop an antibody combination that can substitute the traditional SSC to make up for the deficiency of mass cytometry and bring its multi-parameter synchronous detection into full play, such that mass cytometry can be applied in the immunophenotype detection of hematologic tumors better.

Stern et al. 2017 discloses two plasma membrane staining assays to evaluate mammalian cell size in mass cytometry experiments.

### SUMMARY

The present disclosure aims to provide an antibody combination for substituting a side scatter signal in mass cytometry hematologic tumor immunophenotyping and use thereof to solve the defects in the prior art.

The present disclosure adopts the following technical solutions:
The first aspect of the present disclosure provides an antibody combination for substituting a side scatter signal in mass cytometry hematologic tumor immunophenotyping, including a Lactoferrin antibody and a Lysozyme antibody, the Lactoferrin antibody and the Lysozyme antibody having metal tags respectively, and the metal tags of the Lactoferrin antibody and the Lysozyme antibody being different.

Further, the metal tag is selected from 89Y, 115In, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 157Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 195Pt, 197Au, 198Pt, and 209Bi.

The second aspect of the present disclosure provides use of the antibody combination above in mass cytometry hematologic tumor immunophenotyping.

Further, the following steps are included:
(1) distinguishing a mature granulocyte subset, a monocyte subset and other cell subsets by the Lactoferrin antibody and the Lysozyme antibody;
(2) distinguishing the other cell subsets by a CD45 antibody, including a primitive and juvenile cell subset or/and an abnormal cell subset, a nucleated red blood cell subset, and a lymphocyte subset; and
(3) analyzing expression of antigens of related subsets by other common hematologic tumor immunophenotyping antibodies to determine whether there is abnormal expression of the antigens of related subsets,
where the Lactoferrin antibody, the Lysozyme antibody, the CD45 antibody, and the other common hematologic tumor immunophenotyping antibodies have metal tags respectively, and the metal tags of the antibodies are different.

Furthermore, the metal tag is selected from 89Y, 115In, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 157Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 195Pt, 197Au, 198Pt, and 209Bi.

The third aspect of the present disclosure provides a gating method for mass cytometry hematologic tumor immunophenotyping, including the following steps:
(1) distinguishing a mature granulocyte subset, a monocyte subset and other cell subsets by the Lactoferrin antibody and the Lysozyme antibody;
(2) distinguishing the other cell subsets by a CD45 antibody, including a primitive and juvenile cell subset or/and an abnormal cell subset, a nucleated red blood cell subset, and a lymphocyte subset; and
(3) analyzing expression of antigens of related subsets by other common hematologic tumor immunophenotyping antibodies to determine whether there is abnormal expression of the antigens of related subsets,
where the Lactoferrin antibody, the Lysozyme antibody, the CD45 antibody, and the other common hematologic tumor immunophenotyping antibodies have metal tags respectively, and the metal tags of the antibodies are different.

Further, the metal tag is selected from 89Y, 115In, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 157Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 195Pt, 197Au, 198Pt, and 209Bi.

The fourth aspect of the present disclosure provides a kit for mass cytometry hematologic tumor immunophenotyping, consisting of 43 monoclonal antibodies with metal tags, as shown in the following table:

| No. | Antibody | Metal | No. | Antibody | Metal |
|---|---|---|---|---|---|
| 1 | cCD3 | 89Y | 23 | κ | 160Gd |
| 2 | CD3 | 115ln | 24 | CD99 | 161Dy |
| 3 | cIgM | 139La | 25 | CD10 | 162Dy |
| 4 | CD56 | 141Pr | 26 | Lysozyme | 163Dy |
| 5 | CD22 | 142Nd | 27 | CD64 | 164Dy |
| 6 | CD235ab | 143Nd | 28 | CD2 | 165Ho |
| 7 | CD61 | 144Nd | 29 | CD117 | 166Er |
| 8 | CD23 | 145Nd | 30 | CD1a | 167Er |
| 9 | CD5 | 146Nd | 31 | CD11c | 168Er |
| 10 | CD15 | 147Sm | 32 | CD45 | 169Tm |
| 11 | CD33 | 148Nd | 33 | CD7 | 170Er |
| 12 | MPO | 149Sm | 34 | CD79a | 171Yb |
| 13 | CD14 | 150Nd | 35 | CD38 | 172Yb |
| 14 | λ | 151Eu | 36 | CD138 | 173Yb |
| 15 | CD13 | 152Sm | 37 | CD20 | 174Yb |
| 16 | CD41 | 153Eu | 38 | TdT | 175Lu |
| 17 | Lactoferrin | 154Sm | 39 | HLA-DR | 176Yb |
| 18 | CD123 | 155Gd | 40 | CD300e | 195Pt |
| 19 | CD34 | 156Gd | 41 | CD4 | 197Au |
| 20 | CD71 | 157Gd | 42 | CD8 | 198pt |
| 21 | CD19 | 158Gd | 43 | CD11b | 209Bi |
| 22 | CD9 | 159Tb | - | - | - |

where numbers 1, 3, 12, 14, 17, 23, 26, 34, and 38 are intracellular antibodies, and others are extracellular antibodies.

The fifth aspect of the present disclosure provides use of the kit above in mass cytometry hematologic tumor immunophenotyping.

Further, the following steps are included:
(1) pre-treating a bone marrow sample to remove mature red blood cells in a bone marrow sample;
(2) detecting, by a mass cytometer, expressive abundance of antigens corresponding to 43 antibodies in the bone marrow sample; and
(3) analyzing, by flow cytometry software, according to the expressive abundance of the antigens corresponding to 43 antibodies in the bone marrow sample, the flow cytometry software including Flowjo analysis software, specifically as follows:
   (3.1) distinguishing a mature granulocyte subset, a monocyte subset and other cell subsets by the Lactoferrin antibody and the Lysozyme antibody;
   (3.2) distinguishing the other cell subsets by a CD45 antibody, including a primitive and juvenile cell subset or/and an abnormal cell subset, a nucleated red blood cell subset, and a lymphocyte subset; and
   (3.3) analyzing expression of antigens of related subsets by other antibodies to determine whether there is abnormal expression of the antigens of related subsets.

### The present disclosure has the following beneficial effects:

1. The present disclosure provides the antibody combination for substituting a side scatter signal in mass cytometry hematologic tumor immunophenotyping. The antibody combination consisting of the Lactoferrin antibody and the Lysozyme antibody substitutes the traditional flow cytometry side scatter signal, such that the function of a traditional flow cytometer to detect SSC is realized in the mass cytometer. The antibody combination is applied, in combination with the CD45 antibody, in mass cytometry hematologic tumor immunophenotyping, which can realize the effect of traditional flow cytometry SSC and CD45 two-dimensional plotting. Moreover, combined with other common antibodies for hematologic tumor immunophenotyping, hematologic tumor immunophenotyping can be carried out, the bone marrow cells are divided into large groups and distinguished, and abnormal subsets can be found.
2. The present disclosure provides the gating method for mass cytometry hematologic tumor immunophenotyping. The mature granulocyte subset, the monocyte subset, and other cell subsets are distinguished first using the Lactoferrin antibody and the Lysozyme antibody. Then the other cell subsets are grouped by the CD45 antibody into the primitive and juvenile cell or/abnormal cell subsets, the nucleated red blood cell subset, and the lymphocyte subset. Then the expression of antigens of related subsets is analyzed by other common antibodies for hematologic tumor immunophenotyping to determine whether there is abnormal expression of the antigens of related subsets, realizing mass cytometry hematologic tumor immunophenotyping. According to the present disclosure, the Lactoferrin antibody and the Lysozyme antibody are used for the first time, are combined with a CD45 antibody for two-stage gating strategy, and are combined with a mass cytometer to substitute traditional flow CD45/SSC to distinguish mature granulocytes, monocytes, nucleated red blood cells, lymphocytes, primitive and juvenile cells, and abnormal cell subsets in bone marrow. This overcomes the technical difficulty that the mass cytometry cannot detect SSC in hematologic tumor cell analysis. Combined with the multi-parameter high-throughput characteristics of the mass cytometry, the present disclosure can improve the depth of present hematologic tumor immunophenotyping, and is convenient for clinicians to analyze hematologic tumors according to a traditional flow cytometry mode.
3. The present disclosure provides the kit for mass cytometry hematologic tumor immunophenotyping, consisting of 43 monoclonal antibodies with metal tags. The kit of the present disclosure overcomes the technical difficulty that mass cytometry cannot detect SSC in hematologic tumor cell analysis, realizes the accurate classification of hematologic tumor cells by mass cytometry, and can detect 43 protein markers simultaneously on a single hematologic tumor cell, increasing the sensitivity, accuracy and economy of detection. By testing, the kit of the present disclosure can realize, by just single-tube detection, the effect of the traditional flow cytometer that requires 8-10 tubes for detection, and expands the range and ability of hematologic tumor-related immunophenotype analysis, without single stain control of each channel, without regulating fluorescence compensation, and reduces experimental procedures and sample sizes, laying a foundation for further realization of intelligence and automation of hematologic tumor immunophenotyping. With the aid of the mass cytometer, by using the kit of the present disclosure, the type and nature of hematologic tumor cells can be rapidly and accurately analyzed and the level of positive cells can be determined, which has important guiding significance for prognosis and formulation of clinical therapeutic regimen. Moreover, the detection samples are saved, and more markers can be detected for a single cell at the same time, which also provides more abundant data for the research of hematologic tumors.
4. With the antibody combination, the gating method and the kit of the present disclosure, it is conducive to the use of the mass cytometer to the standardization, normalization, automation and intelligence of the hematologic tumor immunophenotyping.

### BRIEF DESCRIPTION OF THE DRAWINGS

FIGS. 1A-1I show bone marrow cell immunophenotyping of healthy human of Example 1, where:
   in FIG. 1A, Lysozyme and Lactoferrin are used for plotting; the bone marrow sample is divided into three sets, where: the lactoferrin+ and Lysozyme+ cell sets are mature granulocyte subsets, expressing mature granulocyte markers CD33, CD11b, and CD15; with medium-strength Lactoferrin, the Lysozyme+ cell set is a monocyte subset, expressing monocyte markers CD14 and CD64; and the cell sets with low expression of Lactoferrin and Lysozyme are a nucleated red blood cell subset and a lymphocyte subset;
   in FIG. 1B, the Lactoferrin+ and Lysozyme+ cell sets are mature granulocyte subsets, expressing mature granulocyte markers CD13 and CD11b;
   in FIG. 1C, the Lactoferrin+ and Lysozyme+ cell sets are mature granulocyte subsets, expressing mature granulocyte markers CD15 and CD33;
   in FIG. 1D, with medium-strength Lactoferrin, the Lysozyme+ cell set is a monocyte subset, expressing monocyte markers CD14 and CD64;
   in FIG. 1E, the cell set with low expression of Lactoferrin and Lysozyme is gated for a next level using CD45 to obtain a CD45+ lymphocyte subset and a CD45-nucleated red blood cell subset;
   in FIG. 1F, CD45+ lymphocytes are grouped using CD19 and CD3 to obtain CD3+CD19- T cells, CD3-CD19+ B cells, and CD3-CD19- NK cells;
   in FIG. 1G, CD3-CD19+ B cells are grouped using κ and λ to obtain κ+B cells and λ+B cells;
   in FIG. 1H: CD3+CD19- T cells are grouped using CD4 and CD8 to obtain CD3+CD4+T cells and CD3+CD8+T cells; and
   in FIG. 1I, CD3-CD19- NK cells are grouped using CD19 and CD56 to obtain CD3-CD19-CD56+ NK cells.
FIGS. 2A-2I show bone marrow cell immunophenotyping of patients with acute lymphoblastic leukemia of Example 2, where:

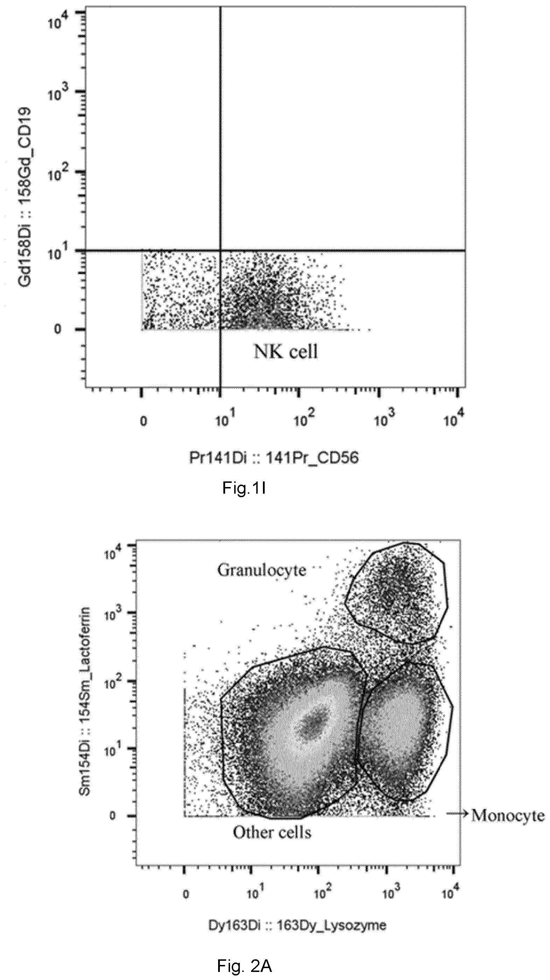

   in FIG. 2A, Lysozyme and Lactoferrin are used for plotting; a bone marrow sample is divided into three sets, where: the lactoferrin+ and Lysozyme+ cell sets are mature granulocyte subsets; with medium-strength Lactoferrin, the Lysozyme+ cell set is a monocyte subset; and the cell sets with low expression of Lactoferrin and Lysozyme are an abnormal cell subset and a lymphocyte subset;

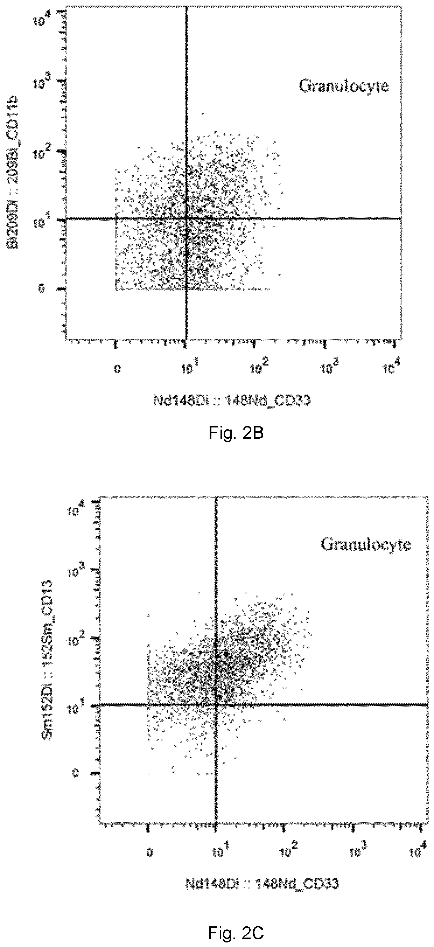

   in FIG. 2B, the Lactoferrin+ and Lysozyme+ cell sets are mature granulocyte subsets, expressing mature granulocyte markers CD33 and CD11b;
   in FIG. 2C, the Lactoferrin+ and Lysozyme+ cell sets are mature granulocyte subsets, expressing mature granulocyte markers CD13 and CD33;

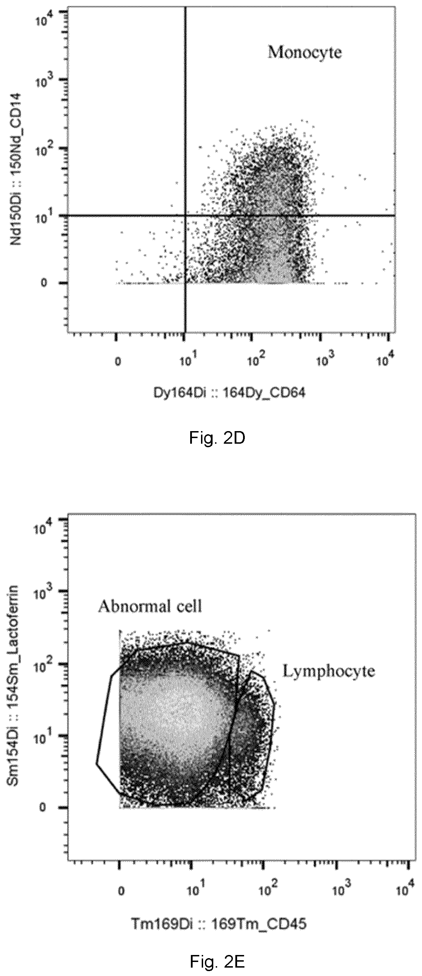

   in FIG. 2D, with medium-strength Lactoferrin, the Lysozyme+ cell set is a monocyte subset, expressing monocyte markers CD14 and CD64;
   in FIG. 2E, the cell set with low expression of Lactoferrin and Lysozyme is gated for a next level using CD45 to obtain a CD45 weakly positive abnormal subset and a CD45+ lymphocyte subset;

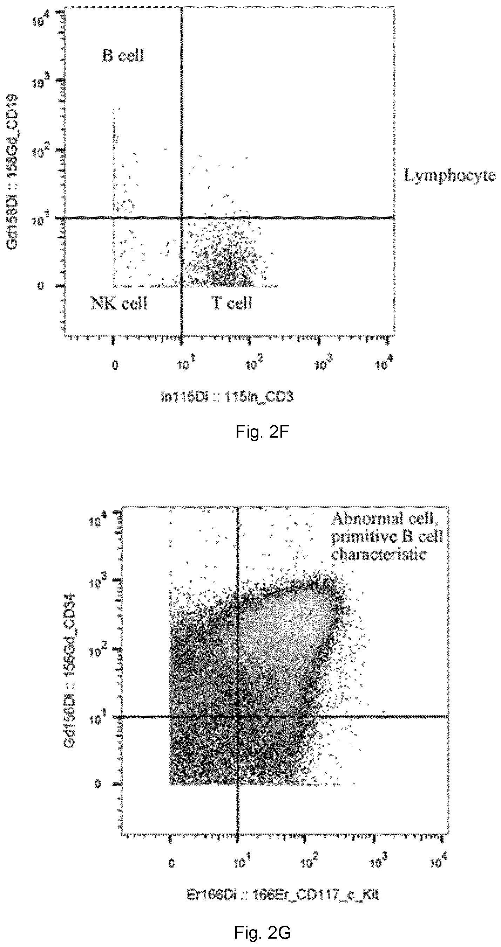

   in FIG. 2F, CD45+ lymphocytes are grouped using CD19 and CD3 to obtain CD3+CD19- T cells, CD3-CD19+ B cells, and CD3-CD19- NK cells;
   in FIG. 2G, the abnormal cell expresses CD34 and CD117, with primitive B cell characteristics;

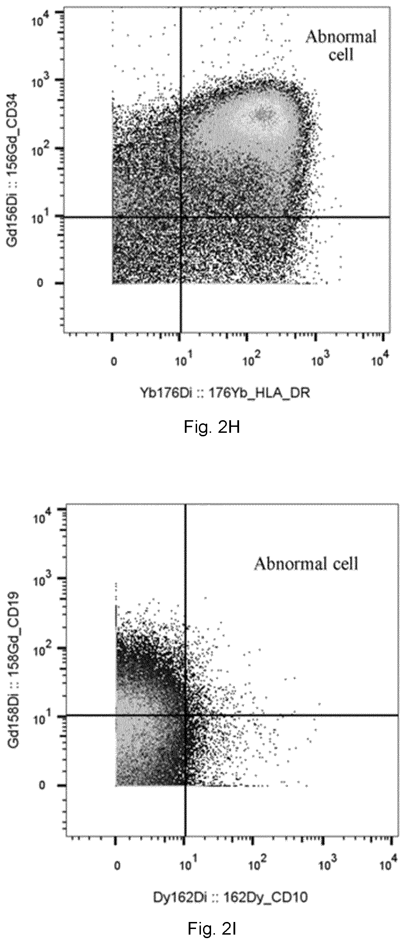

   in FIG. 2H, the abnormal cell expresses CD34 and HLA-DR; and
   in FIG. 2I, the abnormal cell expresses CD19.

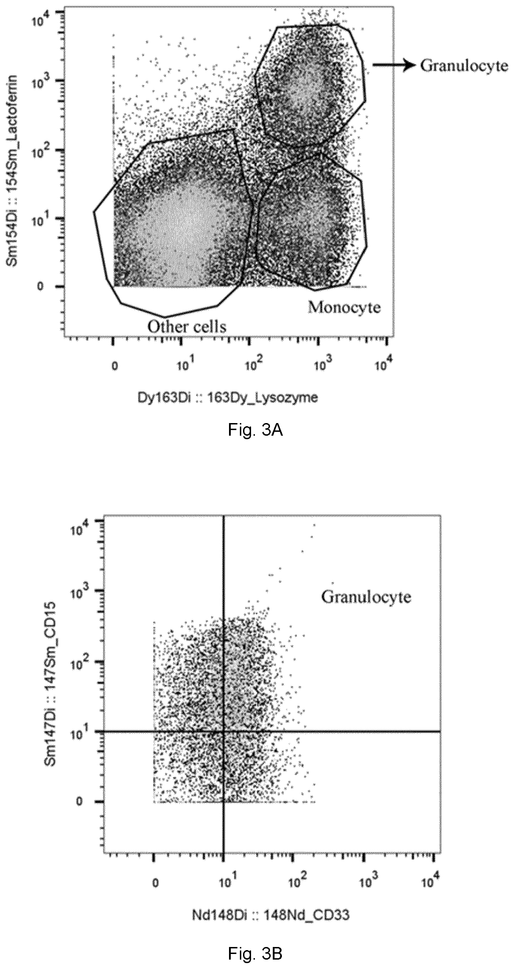

FIGS. 3A-3M show bone marrow cell immunophenotyping of patients with acute myelogenous leukemia of Example 3, where:
   FIG. 3A, Lysozyme and Lactoferrin are used for plotting; a bone marrow sample is divided into three sets, where: the lactoferrin+ and Lysozyme+ cell sets are mature granulocyte subsets; with medium-strength Lactoferrin, the Lysozyme+ cell set is a monocyte subset; and the cell sets with low expression of Lactoferrin and Lysozyme are a nucleated red blood cell subset, an abnormal cell subset and a lymphocyte subset;
   in FIG. 3B, the Lactoferrin+ and Lysozyme+ cell sets are mature granulocyte subsets, expressing mature granulocyte markers CD15 and CD33;

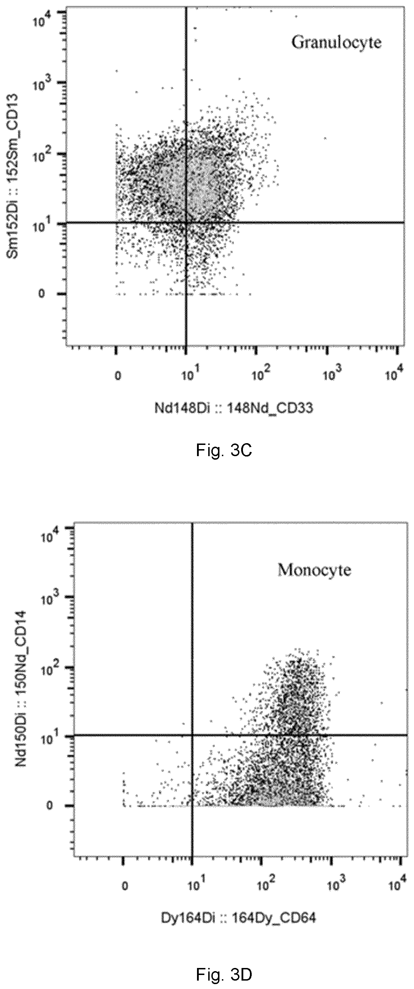

   in FIG. 3C, the Lactoferrin+ and Lysozyme+ cell sets are mature granulocyte subsets, expressing mature granulocyte markers CD13 and CD33;
   in FIG. 3D, with medium-strength Lactoferrin, the Lysozyme+ cell set is a monocyte subset, expressing monocyte markers CD14 and CD64;

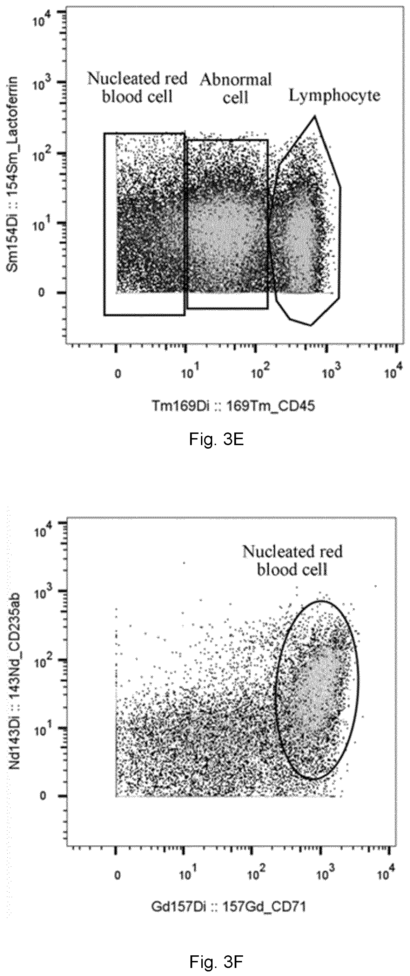

   in FIG. 3E, the cell set with low expression of Lactoferrin and Lysozyme is gated for a next level using CD45 to obtain a CD45+ lymphocyte subset, a CD45 weakly positive abnormal subset and a CD45 negative nucleated red blood cell subset;
   in FIG. 3F, the nucleated red blood cell subset expresses CD71 and CD235ab;

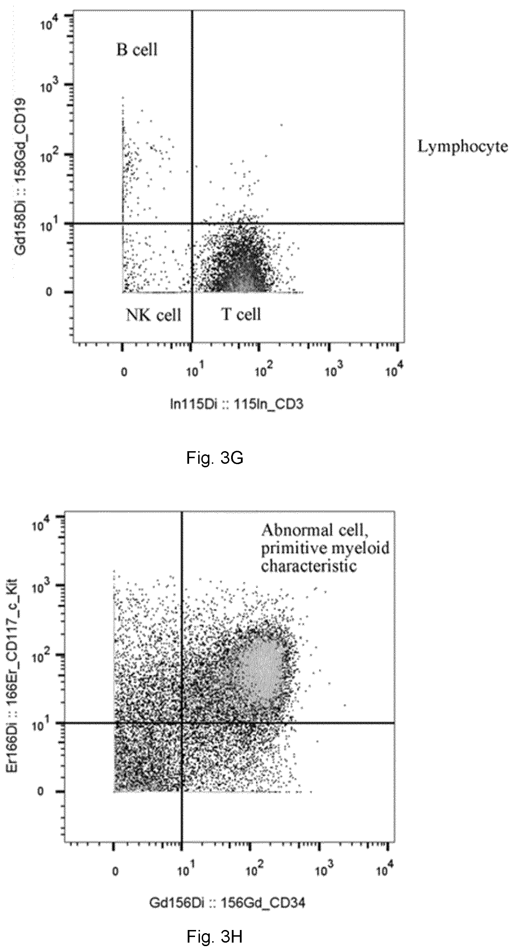

   in FIG. 3G, CD45+ lymphocytes are grouped using CD19 and CD3 to obtain CD3+CD19- T cells, CD3-CD19+ B cells, and CD3-CD19- NK cells;
   in FIG. 3H, the abnormal cell expresses CD34 and CD117;

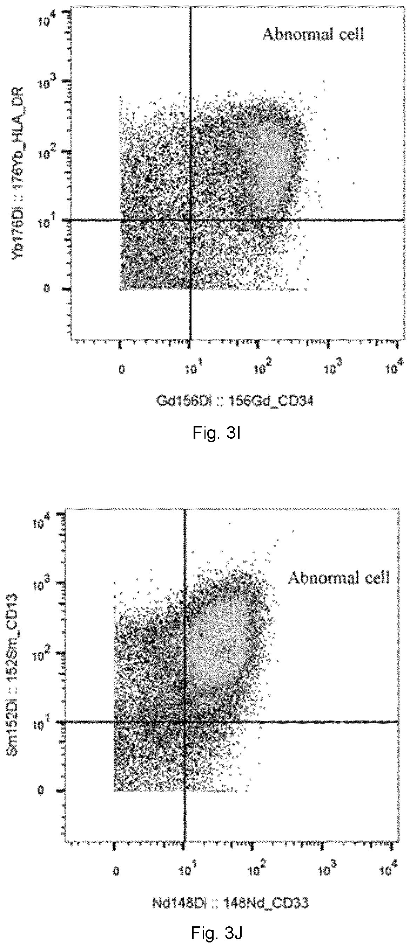

   in FIG. 3I, the abnormal cell expresses CD34 and HLA-DR;
   in FIG. 3J, the abnormal cell expresses CD33 and CD13;

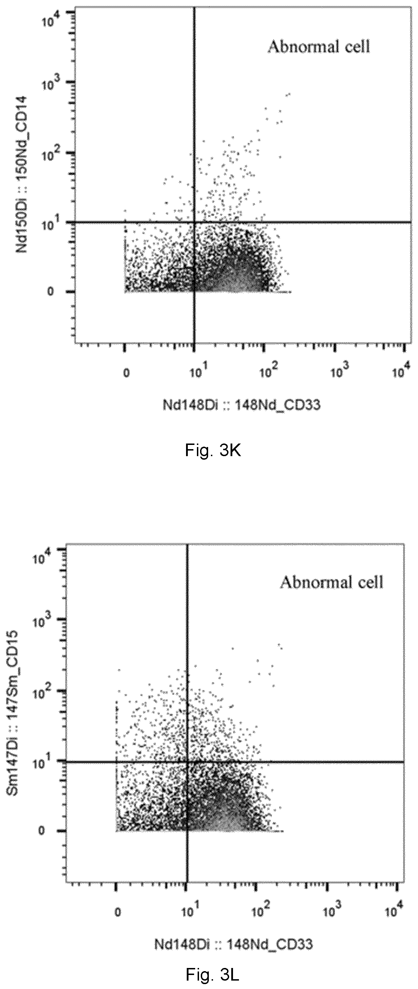

   in FIG. 3K, the abnormal cell expresses CD33, but not express CD14;
   in FIG. 3L, the abnormal cell expresses CD33, but not express CD15; and

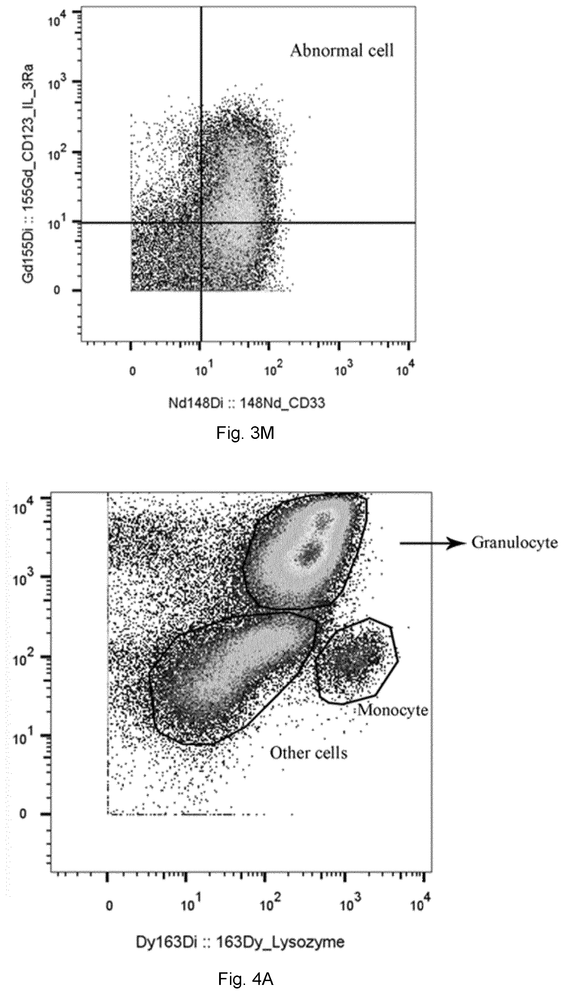

   in FIG. 3M, the abnormal cell expresses CD33 and CD123.
FIGS. 4A-4L show bone marrow cell immunophenotyping of patients with myelodysplastic syndrome of Example 4, where:
   in FIG. 4A, Lysozyme and Lactoferrin are used for plotting; a bone marrow sample is divided into three sets, where: the lactoferrin+ and Lysozyme+ cell sets are mature granulocyte subsets; with medium-strength Lactoferrin, the Lysozyme+ cell set is a monocyte subset; and the cell sets with low expression of Lactoferrin and Lysozyme are an abnormal cell subset and a lymphocyte subset;

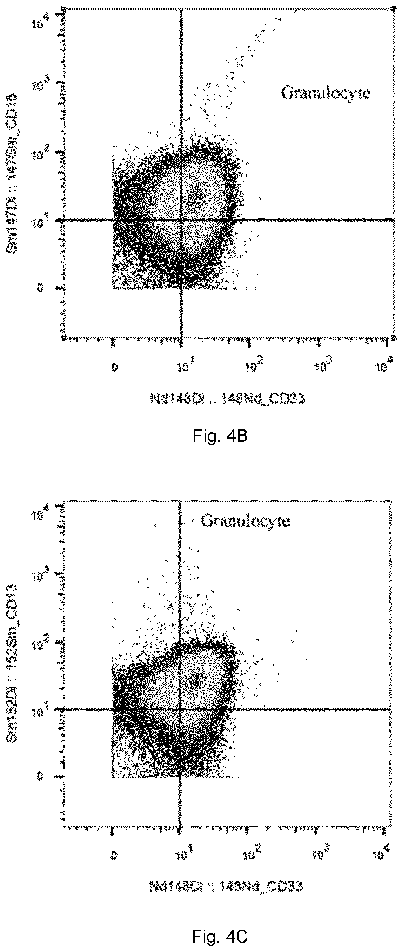

   in FIG. 4B, the Lactoferrin+ and Lysozyme+ cell sets are mature granulocyte subsets, expressing mature granulocyte markers CD15 and CD33;
   in FIG. 4C, the Lactoferrin+ and Lysozyme+ cell sets are mature granulocyte subsets, expressing mature granulocyte markers CD13 and CD33;

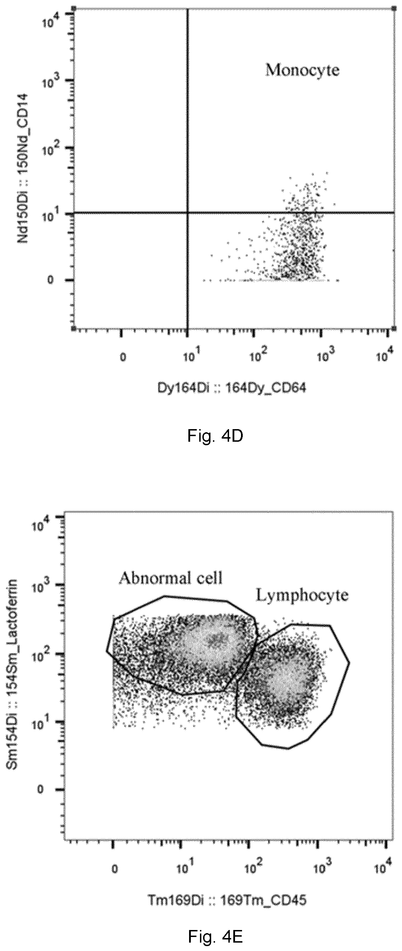

   in FIG. 4D, with medium-strength Lactoferrin, the Lysozyme+ cell set is a monocyte subset, expressing monocyte markers CD14 and CD64;
   in FIG. 4E, the cell set with low expression of Lactoferrin and Lysozyme is gated for a next level using CD45 to obtain a CD45+ lymphocyte subset and a CD45 weakly positive abnormal subset;

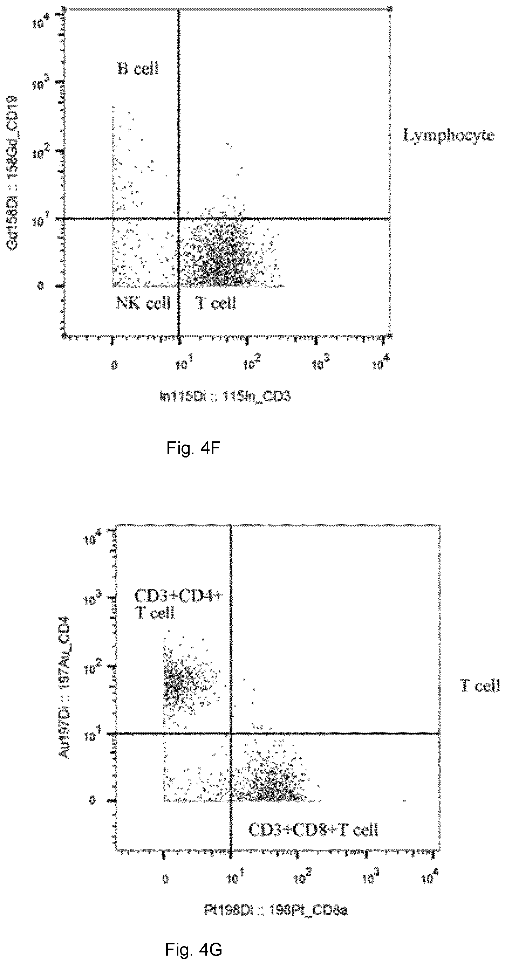

   in FIG. 4F, CD45+ lymphocytes are grouped using CD19 and CD3 to obtain CD3+CD19- T cells, CD3-CD19+ B cells, and CD3-CD19- NK cells;
   in FIG. 4G, CD3-CD19- T cells are grouped using CD4 and CD8 to obtain CD3+CD4+T cells and CD3+CD8+T cells;

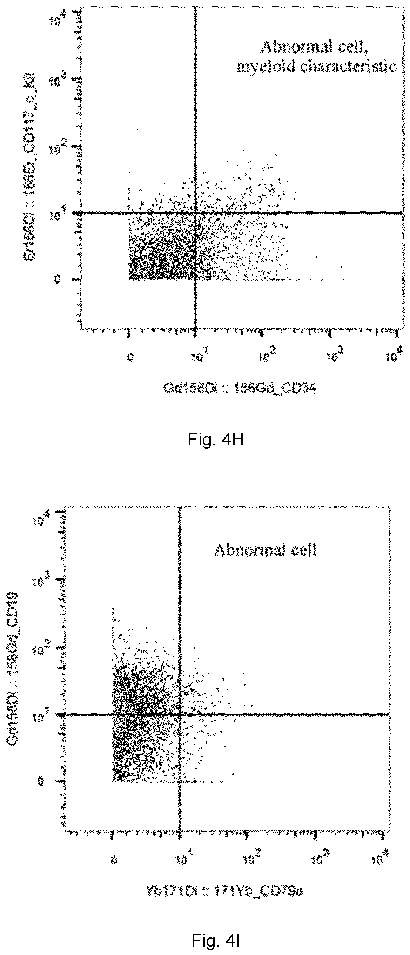

   in FIG. 4H, the abnormal cell does not express CD34 and CD117;
   in FIG. 4I, the abnormal cell expresses CD19, but not express CD79a;

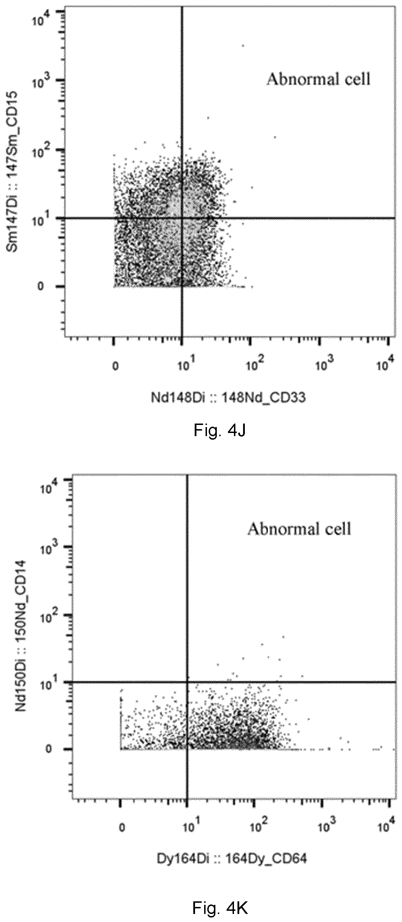

   in FIG. 4J, the abnormal cell expresses CD33 and CD15;
   in FIG. 4K, the abnormal cell expresses CD64, but not express CD14; and

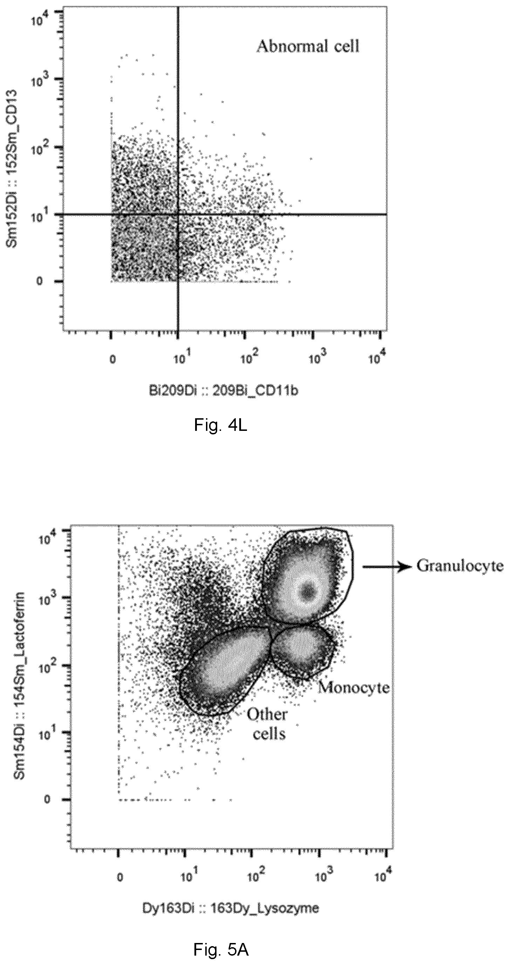

   in FIG. 4L, the abnormal cell expresses CD13, with a small amount of CD11b.
FIGS. 5A-5M show bone marrow cell immunophenotyping of patients with multiple myeloma of Example 5, where:
   in FIG. 5A, Lysozyme and Lactoferrin are used for plotting; a bone marrow sample is divided into three sets, where: the lactoferrin+ and Lysozyme+ cell sets are mature granulocyte subsets; with medium-strength Lactoferrin, the Lysozyme+ cell set is a monocyte subset; and the cell sets with low expression of Lactoferrin and Lysozyme are an abnormal cell subset and a lymphocyte subset;

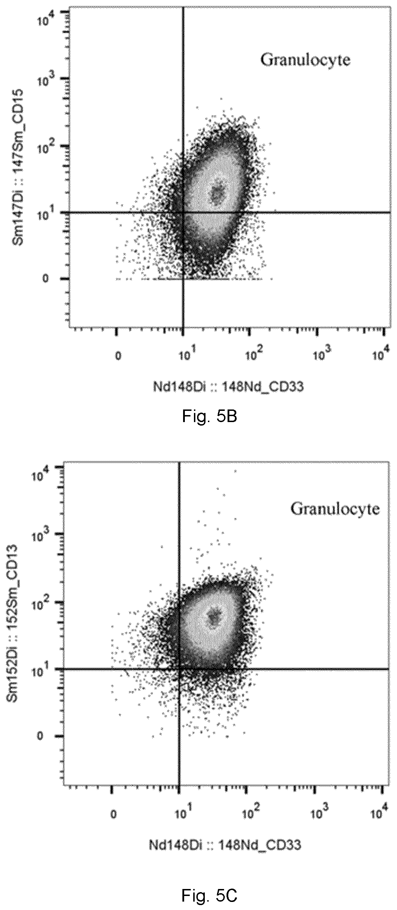

   in FIG. 5B, the Lactoferrin+ and Lysozyme+ cell sets are mature granulocyte subsets, expressing mature granulocyte markers CD15 and CD33;
   in FIG. 5C, the Lactoferrin+ and Lysozyme+ cell sets are mature granulocyte subsets, expressing mature granulocyte markers CD13 and CD33;

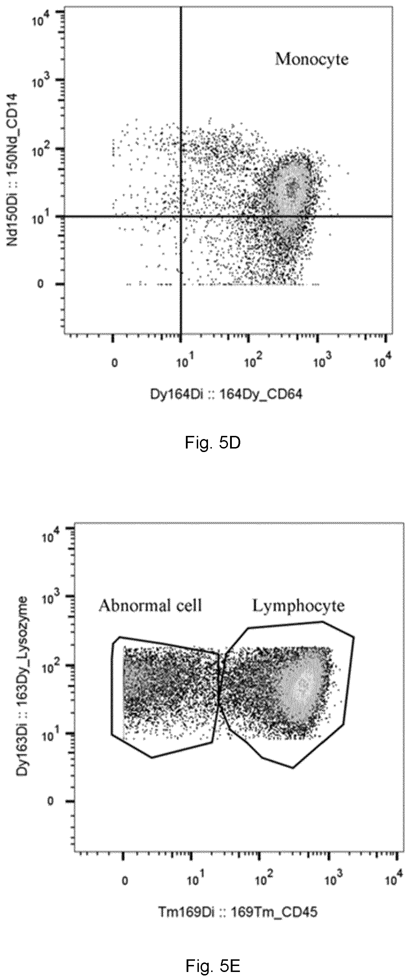

   in FIG. 5D, with medium-strength Lactoferrin, the Lysozyme+ cell set is a monocyte subset, expressing monocyte markers CD14 and CD64;
   in FIG. 5E, the cell set with low expression of Lactoferrin and Lysozyme is gated for a next level using CD45 to obtain a CD45+ lymphocyte subset and a CD45 negative abnormal subset;

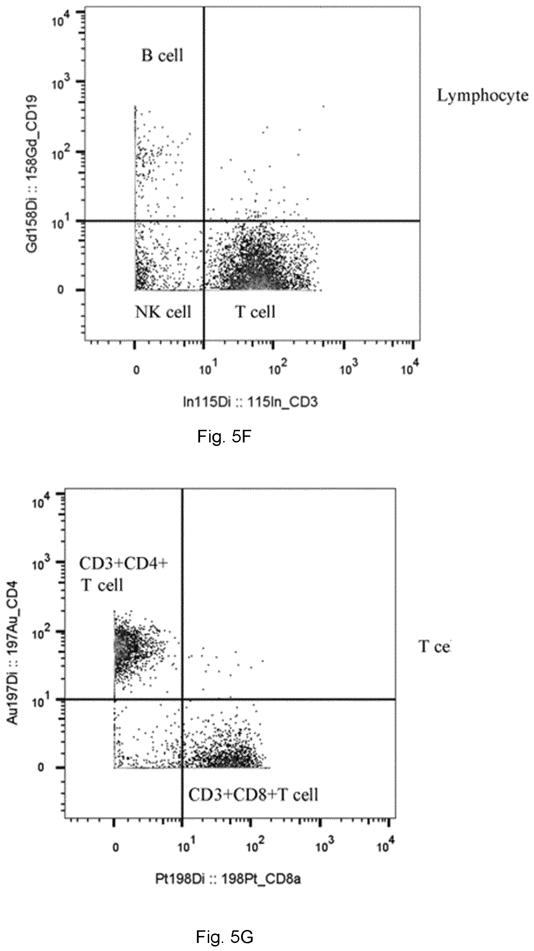

   in FIG. 5F, CD45+ lymphocytes are grouped using CD19 and CD3 to obtain CD3+CD19- T cells, CD3-CD19+ B cells, and CD3-CD19- NK cells;
   in FIG. 5G, CD3-CD19- T cells are grouped using CD4 and CD8 to obtain CD3+CD4+T cells and CD3+CD8+T cells;

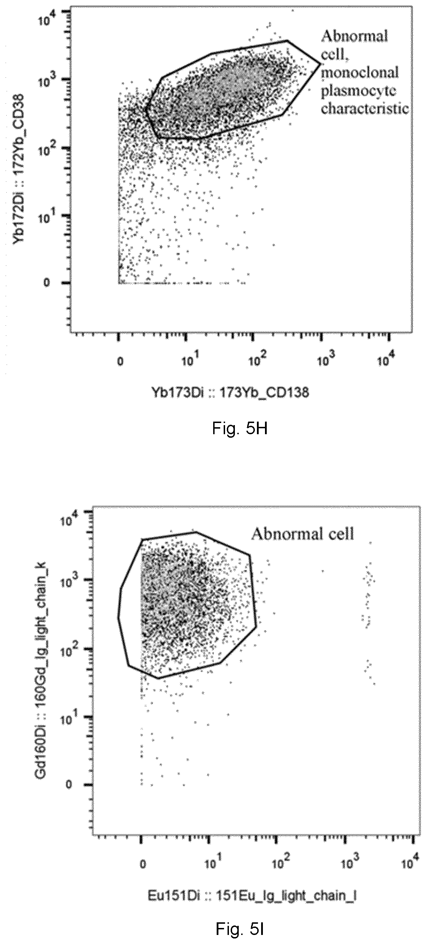

   in FIG. 5H, the abnormal cell expresses CD38 and CD138;
   in FIG. 5I, the abnormal cell expresses κ;

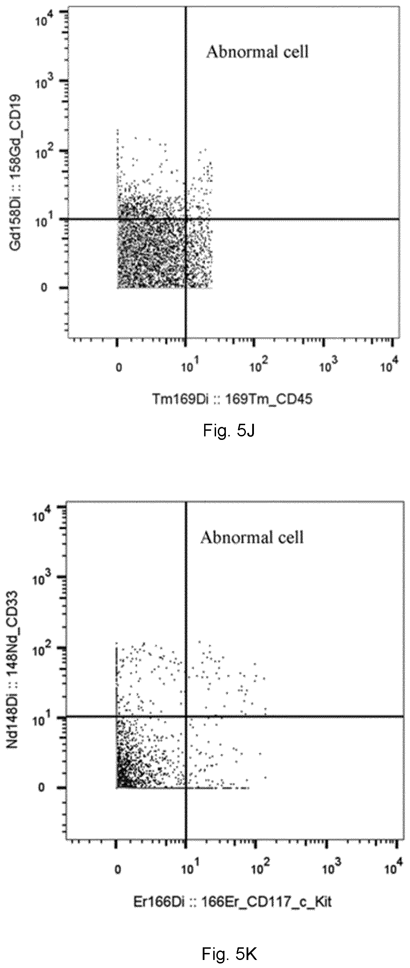

   in FIG. 5J, the abnormal cell does not express CD19 or CD45;
   in FIG. 5K, the abnormal cell does not express CD33 or CD117;

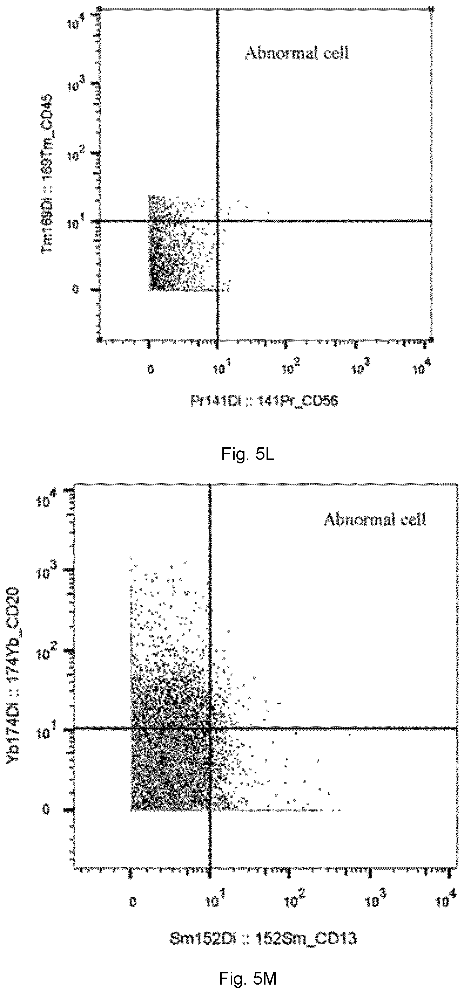

   in FIG. 5L, the abnormal cell does not express CD45 or CD56; and
   in FIG. 5M, the abnormal cell does not express CD13, with a small amount of cells expressing CD20.

### DETAILED DESCRIPTION

The present disclosure will be further explained below in conjunction with the examples and drawings. The following examples are only used to illustrate the present disclosure, but cannot be used to limit the implementation scope of the present disclosure.

The antibodies involved in the following examples are as shown in Table 1:

**Table 1**

| No. | Antibody | Metal | Clone | No. | Antibody | Metal | Clone |
|---|---|---|---|---|---|---|---|
| 1 | cCD3 | 89Y | UCHT1 | 23 | κ | 160Gd | MHK-49 |
| 2 | CD3 | 115ln | UCHT1 | 24 | CD99 | 161Dy | hec2 |
| 3 | cIgM | 139La | MHM-88 | 25 | CD10 | 162Dy | HI10a |
| 4 | CD56 | 141Pr | NCAM16.2 | 26 | Lysozyme | 163Dy | BGN/0696/5B1 |
| 5 | CD22 | 142Nd | HIB22 | 27 | CD64 | 164Dy | 10.1 |
| 6 | CD235ab | 143Nd | HIR2 | 28 | CD2 | 165Ho | RPA-2.10 |
| 7 | CD61 | 144Nd | VI-PL2 | 29 | CD117 | 166Er | 104D2 |
| 8 | CD23 | 145Nd | EBVC5-5 | 30 | CD1a | 167Er | HI149 |
| 9 | CD5 | 146Nd | UCHT2 | 31 | CD11c | 168Er | Bu15 |
| 10 | CD15 | 147Sm | W6D3 | 32 | CD45 | 169Tm | HI30 |
| 11 | CD33 | 148Nd | WM53 | 33 | CD7 | 170Er | CD7-6B7 |
| 12 | MPO | 149Sm | 1B10 | 34 | CD79a | 171Yb | HM47 |
| 13 | CD14 | 150Nd | M5E2 | 35 | CD38 | 172Yb | HIT2 |
| 14 | λ | 151Eu | MHL-38 | 36 | CD138 | 173Yb | DL101 |
| 15 | CD13 | 152Sm | WM15 | 37 | CD20 | 174Yb | 2H7 |
| 16 | CD41 | 153Eu | HIP-8 | 38 | TdT | 175Lu | 4B10A6 |
| 17 | Lactoferrin | 154Sm | 1C6 | 39 | HLA-DR | 176Yb | L243 |
| 18 | CD123 | 155Gd | 6H6 | 40 | CD300e | 195Pt | UP-H2 |
| 19 | CD34 | 156Gd | 581 | 41 | CD4 | 197Au | RPA-T4 |
| 20 | CD71 | 157Gd | CY1G4 | 42 | CD8 | 198pt | RPA-T8 |
| 21 | CD19 | 158Gd | HIB19 | 43 | CD11b | 209Bi | M1/70 |
| 22 | CD9 | 159Tb | SN4 C3-3A2 | - | - | - | |

where cCD3, cIgM, MPO, λ, Lactoferrin, κ, Lysozyme, CD79a, and TdT antibodies with numbers 1, 3, 12, 14, 17, 23, 26, 34, and 38 are intracellular antibodies, and others are extracellular antibodies.

### Example 1: bone marrow cell immunophenotyping of healthy human

1) Fresh bone marrow of healthy human was prepared, with mature red blood cells removed.
2) 1-3x10^6 cells were taken and re-suspended with PBS, the volume was adjusted to 1 mL, 50 µL-1 mL of 194Pt (0.1-1 µM) was added, and staining was carried out at room temperature for 2 min to determine whether the cells were dead or alive.
3) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, and 50 µL of blocking buffers was added for blocking on ice for 20 min. The blocking buffer consisted of 0.5 µL of human immunoglobulin solutions (including 15-25 parts by mass of human immunoglobulin, 0.15-0.25 parts by mass of sodium azide, and 0.75-1.25 parts by volume of phosphate buffers), 0.5 µL of mouse immunoglobulin solutions (including 15-25 parts by mass of mouse immunoglobulin, 0.15-0.25 parts by mass of sodium azide, and 0.75-1.25 parts by volume of phosphate buffers), 0.5 µL of rat immunoglobulin solutions (including 15-25 parts by mass of rat immunoglobulin, 0.15-0.25 parts by mass of sodium azide, and 0.75-1.25 parts by volume of phosphate buffers), 0.5 µL of hamster immunoglobulin solutions (including 15-25 parts by mass of hamster immunoglobulin, 0.15-0.25 parts by mass of sodium azide, and 0.75-1.25 parts by volume of phosphate buffers), and 48 µL of bovine serum albumin solutions (including 375-625 parts by mass of bovine serum albumin, 15-25 parts by mass of sodium azide, and 75-125 parts by volume of phosphate buffers).
4) 50 µL of extracellular antibody mixed liquid (0.5 µL of each of 34 extracellular antibodies in Table 1, at an antibody concentration of 0.1-1 µg/µL respectively, and 33 µL of bovine serum albumin solutions (including 375-625 parts by mass of bovine serum albumin, 15-25 parts by mass of sodium azide, and 75-125 parts by volume of phosphate buffers) was added, cells were resuspended, and staining was carried out on ice for 30 min.
5) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, 1 mL of fixation/permeabilization solutions containing 0.5 v/v‰ single-cell indicator 191/193 Ir was added and cells were re-suspended overnight at 4°C.
6) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction, for the control group, 50 µL of fixation-permeabilization solutions was added as blank control, for the experimental group, 50 µL of intracellular antibody mixed liquid (0.5 µL of each of 9 intracellular antibodies in Table 1, at an antibody concentration of 0.1-1 µg/µL respectively, and 45.5 µL of bovine serum albumin solutions (including 375-625 parts by mass of bovine serum albumin, 15-25 parts by mass of sodium azide, and 75-125 parts by volume of phosphate buffers)) was added, cells were suspended and placed on ice for 30 min.
7) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
8) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
9) 2 mL of deionized water was added and centrifuged at 800 g/5 min, and supernatant was removed by suction.
10) 2 mL of deionized water was added and centrifuged at 800 g/5 min, and supernatant was removed by suction.
11) The sample was filtered, the cells were counted, the volume was adjusted, and preparation was carried out for on-machine mass cytometry detection.

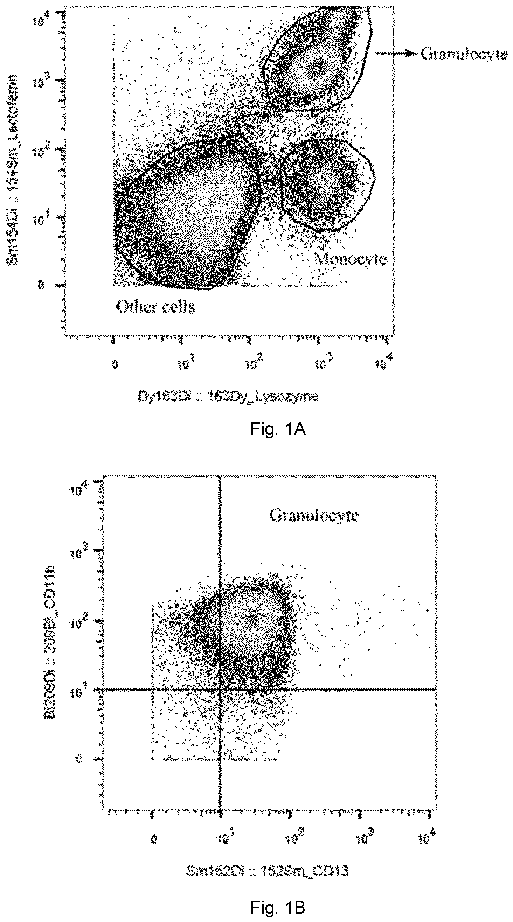

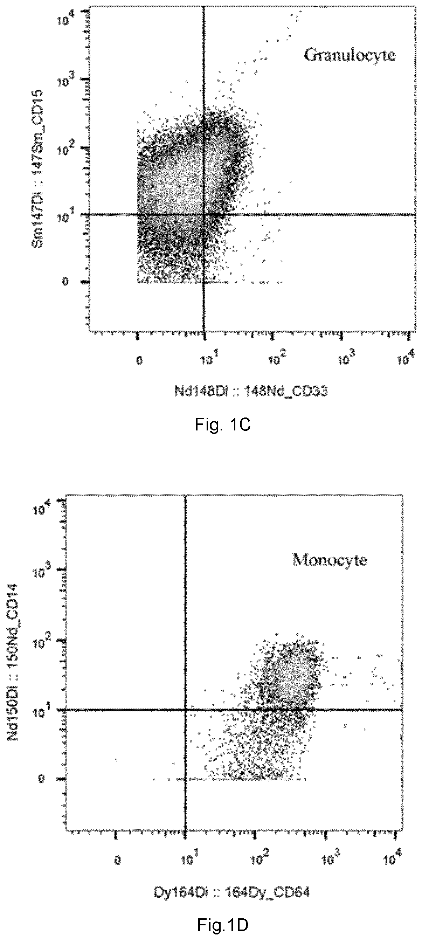

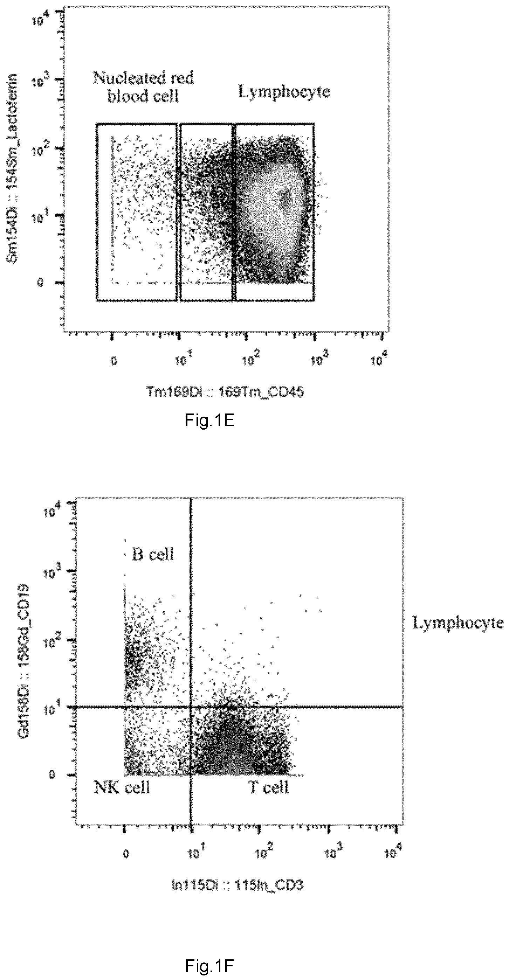

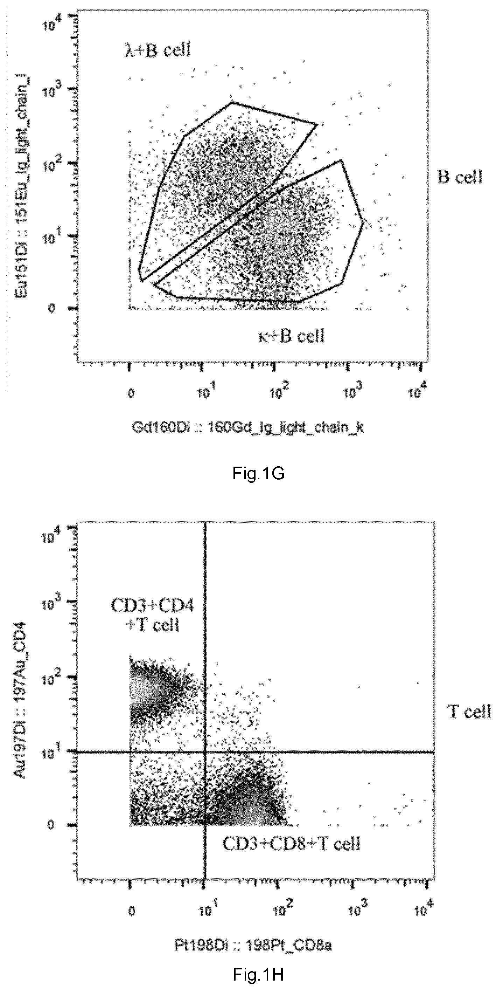

The analysis results are shown in FIG. 1A. Lysozyme and Lactoferrin are used for plotting. The bone marrow sample is divided into three sets: the lactoferrin+ and Lysozyme+ cell sets are mature granulocyte subsets, expressing mature granulocyte markers CD33, CD11b, and CD15 (FIGS. 1B and 1C); with medium-strength Lactoferrin, the Lysozyme+ cell set is a monocyte subset, expressing monocyte markers CD14 and CD64 (FIG. 1D); and the cell sets with low expression of Lactoferrin and Lysozyme are a nucleated red blood cell subset and a lymphocyte subset. As shown in FIG. 1E, cell set with low expression of Lactoferrin and Lysozyme is gated for a next level using CD45 to obtain a CD45+ lymphocyte subset and a CD45-nucleated red blood cell subset. As shown in FIG. 1F, CD45+ lymphocytes are grouped using CD19 and CD3 to obtain CD3+CD19- T cells (FIG. 1H), CD3-CD19+ B cells (FIG. 1G), and CD3-CD19- NK cells (FIG. 1I).

### Example 2: bone marrow cell immunophenotyping of patients with acute lymphoblastic leukemia

1) Fresh bone marrow of patients with acute lymphoblastic leukemia was prepared, with mature red blood cells removed.
2) 1-3x10^6 cells were taken and re-suspended with PBS, the volume was adjusted to 1 mL, 50 µL-1 mL of 194Pt (0.1-1 µM) was added, and staining was carried out at room temperature for 2 min to determine whether the cells were dead or alive.
3) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, and 50 µL of blocking buffers was added for blocking on ice for 20 min. The blocking buffer consisted of 0.5 µL of human immunoglobulin solutions (including 15-25 parts by mass of human immunoglobulin, 0.15-0.25 parts by mass of sodium azide, and 0.75-1.25 parts by volume of phosphate buffers), 0.5 µL of mouse immunoglobulin solutions (including 15-25 parts by mass of mouse immunoglobulin, 0.15-0.25 parts by mass of sodium azide, and 0.75-1.25 parts by volume of phosphate buffers), 0.5 µL of rat immunoglobulin solutions (including 15-25 parts by mass of rat immunoglobulin, 0.15-0.25 parts by mass of sodium azide, and 0.75-1.25 parts by volume of phosphate buffers), 0.5 µL of hamster immunoglobulin solutions (including 15-25 parts by mass of hamster immunoglobulin, 0.15-0.25 parts by mass of sodium azide, and 0.75-1.25 parts by volume of phosphate buffers), and 48 µL of bovine serum albumin solutions (including 375-625 parts by mass of bovine serum albumin, 15-25 parts by mass of sodium azide, and 75-125 parts by volume of phosphate buffers).
4) 50 µL of extracellular antibody mixed liquid (0.5 µL of each of 34 extracellular antibodies in Table 1, at an antibody concentration of 0.1-1 µg/µL respectively, and 33 µL of bovine serum albumin solutions (including 375-625 parts by mass of bovine serum albumin, 15-25 parts by mass of sodium azide, and 75-125 parts by volume of phosphate buffers) was added, cells were resuspended, and staining was carried out on ice for 30 min.
5) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, 1 mL of fixation/permeabilization solutions containing 0.5 v/v‰ single-cell indicator 191/193 Ir was added and cells were re-suspended overnight at 4°C.
6) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction, for the control group, 50 µL of fixation-permeabilization solutions was added as blank control, for the experimental group, 50 µL of intracellular antibody mixed liquid (0.5 µL of each of 9 intracellular antibodies in Table 1, at an antibody concentration of 0.1-1 µg/µL respectively, and 45.5 µL of bovine serum albumin solutions (including 375-625 parts by mass of bovine serum albumin, 15-25 parts by mass of sodium azide, and 75-125 parts by volume of phosphate buffers)) was added, cells were suspended and placed on ice for 30 min.
7) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
8) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
9) 2 mL of deionized water was added and centrifuged at 800 g/5 min, and supernatant was removed by suction.
10) 2 mL of deionized water was added and centrifuged at 800 g/5 min, and supernatant was removed by suction.
11) The sample was filtered, the cells were counted, the volume was adjusted, and preparation was carried out for on-machine mass cytometry detection.

The analysis results are shown in FIG. 2A. Lysozyme and Lactoferrin are used for plotting. The bone marrow sample is divided into three sets: the lactoferrin+ and Lysozyme+ cell sets are mature granulocyte subsets (FIGS. 2B and 2C); with medium-strength Lactoferrin, the Lysozyme+ cell set is a monocyte subset (FIG. 2D); and the cell sets with low expression of Lactoferrin and Lysozyme are an abnormal cell subset and a lymphocyte subset. As shown in FIG. 2E, the cell set with low expression of Lactoferrin and Lysozyme is gated for a next level using CD45 to obtain a CD45+ lymphocyte subset (FIG. 2F) and a CD45 weakly positive abnormal cell subset. The abnormal cell expresses CD34, CD117, HLA-DR, and CD19 (FIGS. 2G, 2H and 2I).

### Example 3: bone marrow cell immunophenotyping of patients with acute myelogenous leukemia

1) Fresh bone marrow of patients with acute myelogenous leukemia was prepared, with mature red blood cells removed.
2) 1-3x10^6 cells were taken and re-suspended with PBS, the volume was adjusted to 1 mL, 50 µL-1 mL of 194Pt (0.1-1 µM) was added, and staining was carried out at room temperature for 2 min to determine whether the cells were dead or alive.
3) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, and 50 µL of blocking buffers was added for blocking on ice for 20 min. The blocking buffer consisted of 0.5 µL of human immunoglobulin solutions (including 15-25 parts by mass of human immunoglobulin, 0.15-0.25 parts by mass of sodium azide, and 0.75-1.25 parts by volume of phosphate buffers), 0.5 µL of mouse immunoglobulin solutions (including 15-25 parts by mass of mouse immunoglobulin, 0.15-0.25 parts by mass of sodium azide, and 0.75-1.25 parts by volume of phosphate buffers), 0.5 µL of rat immunoglobulin solutions (including 15-25 parts by mass of rat immunoglobulin, 0.15-0.25 parts by mass of sodium azide, and 0.75-1.25 parts by volume of phosphate buffers), 0.5 µL of hamster immunoglobulin solutions (including 15-25 parts by mass of hamster immunoglobulin, 0.15-0.25 parts by mass of sodium azide, and 0.75-1.25 parts by volume of phosphate buffers), and 48 µL of bovine serum albumin solutions (including 375-625 parts by mass of bovine serum albumin, 15-25 parts by mass of sodium azide, and 75-125 parts by volume of phosphate buffers).
4) 50 µL of extracellular antibody mixed liquid (0.5 µL of each of 34 extracellular antibodies in Table 1, at an antibody concentration of 0.1-1 µg/µL respectively, and 33 µL of bovine serum albumin solutions (including 375-625 parts by mass of bovine serum albumin, 15-25 parts by mass of sodium azide, and 75-125 parts by volume of phosphate buffers) was added, cells were resuspended, and staining was carried out on ice for 30 min.
5) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, 1 mL of fixation/permeabilization solutions containing 0.5 v/v‰ single-cell indicator 191/193 Ir was added and cells were re-suspended overnight at 4°C.
6) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction, for the control group, 50 µL of fixation-permeabilization solutions was added as blank control, for the experimental group, 50 µL of intracellular antibody mixed liquid (0.5 µL of each of 9 intracellular antibodies in Table 1, at an antibody concentration of 0.1-1 µg/µL respectively, and 45.5 µL of bovine serum albumin solutions (including 375-625 parts by mass of bovine serum albumin, 15-25 parts by mass of sodium azide, and 75-125 parts by volume of phosphate buffers)) was added, cells were suspended and placed on ice for 30 min.
7) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
8) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
9) 2 mL of deionized water was added and centrifuged at 800 g/5 min, and supernatant was removed by suction.
10) 2 mL of deionized water was added and centrifuged at 800 g/5 min, and supernatant was removed by suction.
11) The sample was filtered, the cells were counted, the volume was adjusted, and preparation was carried out for on-machine mass cytometry detection.

The analysis results are shown in FIG. 3A. Lysozyme and Lactoferrin are used for plotting. The bone marrow sample is divided into three sets: the lactoferrin+ and Lysozyme+ cell sets are mature granulocyte subsets (FIGS. 3B and 3C); with medium-strength Lactoferrin, the Lysozyme+ cell set is a monocyte subset (FIG. 3D); and the cell sets with low expression of Lactoferrin and Lysozyme are a nucleated red blood cell subset, an abnormal cell subset and a lymphocyte subset. As shown in FIG. 3E, the cell set with low expression of Lactoferrin and Lysozyme is gated for a next level using CD45 to obtain a CD45+ lymphocyte subset (FIG. 3G), a CD45 weakly positive abnormal cell subset, and a CD45 negative nucleated red blood cell subset (FIG. 3F). The abnormal cell expresses CD34, CD117, HLA-DR, CD33, CD13, and CD123 (FIGS. 3H, 3I, 3J, 3K, 3L and 3M).

### Example 4: bone marrow cell immunophenotyping of patients with myelodysplastic syndrome (MDS)

1) Fresh bone marrow of patients with myelodysplastic syndrome was prepared, with mature red blood cells removed.
2) 1-3x10^6 cells were taken and re-suspended with PBS, the volume was adjusted to 1 mL, 50 µL-1 mL of 194Pt (0.1-1 µM) was added, and staining was carried out at room temperature for 2 min to determine whether the cells were dead or alive.
3) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, and 50 µL of blocking buffers was added for blocking on ice for 20 min. The blocking buffer consisted of 0.5 µL of human immunoglobulin solutions (including 15-25 parts by mass of human immunoglobulin, 0.15-0.25 parts by mass of sodium azide, and 0.75-1.25 parts by volume of phosphate buffers), 0.5 µL of mouse immunoglobulin solutions (including 15-25 parts by mass of mouse immunoglobulin, 0.15-0.25 parts by mass of sodium azide, and 0.75-1.25 parts by volume of phosphate buffers), 0.5 µL of rat immunoglobulin solutions (including 15-25 parts by mass of rat immunoglobulin, 0.15-0.25 parts by mass of sodium azide, and 0.75-1.25 parts by volume of phosphate buffers), 0.5 µL of hamster immunoglobulin solutions (including 15-25 parts by mass of hamster immunoglobulin, 0.15-0.25 parts by mass of sodium azide, and 0.75-1.25 parts by volume of phosphate buffers), and 48 µL of bovine serum albumin solutions (including 375-625 parts by mass of bovine serum albumin, 15-25 parts by mass of sodium azide, and 75-125 parts by volume of phosphate buffers).
4) 50 µL of extracellular antibody mixed liquid (0.5 µL of each of 34 extracellular antibodies in Table 1, at an antibody concentration of 0.1-1 µg/µL respectively, and 33 µL of bovine serum albumin solutions (including 375-625 parts by mass of bovine serum albumin, 15-25 parts by mass of sodium azide, and 75-125 parts by volume of phosphate buffers) was added, cells were resuspended, and staining was carried out on ice for 30 min.
5) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, 1 mL of fixation/permeabilization solutions containing 0.5 v/v‰ single-cell indicator 191/193 Ir was added and cells were re-suspended overnight at 4°C.
6) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction, for the control group, 50 µL of fixation-permeabilization solutions was added as blank control, for the experimental group, 50 µL of intracellular antibody mixed liquid (0.5 µL of each of 9 intracellular antibodies in Table 1, at an antibody concentration of 0.1-1 µg/µL respectively, and 45.5 µL of bovine serum albumin solutions (including 375-625 parts by mass of bovine serum albumin, 15-25 parts by mass of sodium azide, and 75-125 parts by volume of phosphate buffers)) was added, cells were suspended and placed on ice for 30 min.
7) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
8) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
9) 2 mL of deionized water was added and centrifuged at 800 g/5 min, and supernatant was removed by suction.
10) 2 mL of deionized water was added and centrifuged at 800 g/5 min, and supernatant was removed by suction.
11) The sample was filtered, the cells were counted, the volume was adjusted, and preparation was carried out for on-machine mass cytometry detection.

The analysis results are shown in FIG. 4A. Lysozyme and Lactoferrin are used for plotting. The bone marrow sample is divided into three sets: the lactoferrin+ and Lysozyme+ cell sets are mature granulocyte subsets (FIGS. 4B and 4C); with medium-strength Lactoferrin, the Lysozyme+ cell set is a monocyte subset (FIG. 4D); and the cell sets with low expression of Lactoferrin and Lysozyme are an abnormal cell subset and a lymphocyte subset. As shown in FIG. 4E, cell set with low expression of Lactoferrin and Lysozyme is gated for a next level using CD45 to obtain a CD45+ lymphocyte subset (FIGS. 4F and 4G) and a CD45 weakly positive abnormal cell subset. The abnormal cell expresses CD33, CD15, CD13, CD11b, CD19, and CD64 (FIGS. 4H, 4I, 4J, 4K and 4L).

### Example 5: bone marrow cell immunophenotyping of patients with multiple myeloma

1) Fresh bone marrow of patients with multiple myeloma was prepared, with mature red blood cells removed.
2) 1-3x10^6 cells were taken and re-suspended with PBS, the volume was adjusted to 1 mL, 50 µL-1 mL of 194Pt (0.1-1 µM) was added, and staining was carried out at room temperature for 2 min to determine whether the cells were dead or alive.
3) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, and 50 µL of blocking buffers was added for blocking on ice for 20 min. The blocking buffer consisted of 0.5 µL of human immunoglobulin solutions (including 15-25 parts by mass of human immunoglobulin, 0.15-0.25 parts by mass of sodium azide, and 0.75-1.25 parts by volume of phosphate buffers), 0.5 µL of mouse immunoglobulin solutions (including 15-25 parts by mass of mouse immunoglobulin, 0.15-0.25 parts by mass of sodium azide, and 0.75-1.25 parts by volume of phosphate buffers), 0.5 µL of rat immunoglobulin solutions (including 15-25 parts by mass of rat immunoglobulin, 0.15-0.25 parts by mass of sodium azide, and 0.75-1.25 parts by volume of phosphate buffers), 0.5 µL of hamster immunoglobulin solutions (including 15-25 parts by mass of hamster immunoglobulin, 0.15-0.25 parts by mass of sodium azide, and 0.75-1.25 parts by volume of phosphate buffers), and 48 µL of bovine serum albumin solutions (including 375-625 parts by mass of bovine serum albumin, 15-25 parts by mass of sodium azide, and 75-125 parts by volume of phosphate buffers).
4) 50 µL of extracellular antibody mixed liquid (0.5 µL of each of 34 extracellular antibodies in Table 1, at an antibody concentration of 0.1-1 µg/µL respectively, and 33 µL of bovine serum albumin solutions (including 375-625 parts by mass of bovine serum albumin, 15-25 parts by mass of sodium azide, and 75-125 parts by volume of phosphate buffers) was added, cells were resuspended, and staining was carried out on ice for 30 min.
5) 2 mL of bovine serum albumin solutions (including 375-625 parts by mass of bovine serum albumin, 15-25 parts by mass of sodium azide, and 75-125 parts by volume of phosphate buffers) was added and centrifuged at 500 g/5 min, supernatant was removed by suction, 1 mL of fixation/permeabilization solutions containing 0.5 v/v‰ single-cell indicator 191/193 Ir was added and cells were re-suspended overnight at 4°C.
6) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction, for the control group, 50 µL of fixation-permeabilization solutions was added as blank control, for the experimental group, 50 µL of intracellular antibody mixed liquid (0.5 µL of each of 9 intracellular antibodies in Table 1, at an antibody concentration of 0.1-1 µg/µL respectively, and 45.5 µL of bovine serum albumin solutions (including 375-625 parts by mass of bovine serum albumin, 15-25 parts by mass of sodium azide, and 75-125 parts by volume of phosphate buffers)) was added, cells were suspended and placed on ice for 30 min.
7) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
8) 2 mL of bovine serum albumin solutions (including 375-625 parts by mass of bovine serum albumin, 15-25 parts by mass of sodium azide, and 75-125 parts by volume of phosphate buffers) was added and centrifuged at 800 g/5 min, and supernatant was removed by suction.
9) 2 mL of deionized water was added and centrifuged at 800 g/5 min, and supernatant was removed by suction.
10) 2 mL of deionized water was added and centrifuged at 800 g/5 min, and supernatant was removed by suction.
11) The sample was filtered, the cells were counted, the volume was adjusted, and preparation was carried out for on-machine mass cytometry detection.

The analysis results are shown in FIG. 5A. Lysozyme and Lactoferrin are used for plotting. The bone marrow sample is divided into three sets: the lactoferrin+ and Lysozyme+ cell sets are mature granulocyte subsets (FIGS. 5B and 5C); with medium-strength Lactoferrin, the Lysozyme+ cell set is a monocyte subset (FIG. 5D); and the cell sets with low expression of Lactoferrin and Lysozyme are an abnormal cell subset and a lymphocyte subset. As shown in FIG. 5E cell set with low expression of Lactoferrin and Lysozyme is gated for a next level using CD45 to obtain a CD45+ lymphocyte subset and a CD45 negative abnormal cell subset (FIGS. 5F and 5G). abnormal cell expresses CD38, CD138, κ, and CD20; and CD56 and CD45 are negative (FIGS. 5H, 5I, 5J, 5K, 5L and 5M).

## Claims

1. A gating method for mass cytometry hematologic tumor immunophenotyping, including the following steps:
(1) distinguishing a mature granulocyte subset, a monocyte subset and other cell subsets by a Lactoferrin antibody and a Lysozyme antibody;
(2) distinguishing the other cell subsets by a CD45 antibody, including a primitive and juvenile cell subset or/and an abnormal cell subset, a nucleated red blood cell subset, and a lymphocyte subset; and
(3) analyzing expression of antigens of the subsets by other common hematologic tumor immunophenotyping antibodies to determine whether there is abnormal expression of the antigens of related subsets,
where the Lactoferrin antibody, the Lysozyme antibody, the CD45 antibody, and the other common hematologic tumor immunophenotyping antibodies have metal tags respectively, and the metal tags of the antibodies are different.

2. The method according to claim 1, wherein the respective metal tag is selected from 89Y, 115In, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 157Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 195Pt, 197Au, 198Pt, and 209Bi.

## Patentansprüche

1. Gating-Verfahren für die hämatologische Tumor-Immunphänotypisierung mittels Massenzytometrie, das die folgenden Schritte umfasst:
(1) Unterscheidung einer reifen Granulozyten-Untergruppe, einer Monozyten-Untergruppe und anderer Zell-Untergruppen durch einen Lactoferrin-Antikörper und einen Lysozym-Antikörper;
(2) Unterscheidung der anderen Zell-Untergruppen durch einen CD45-Antikörper, einschließlich einer primitiven und juvenilen Zell-Untergruppe oder/und einer abnormalen Zell-Untergruppe, einer kernhaltigen roten Blutkörperchen-Untergruppe und einer Lymphozyten-Untergruppe; und
(3) Analysieren der Expression von Antigenen der Untergruppen durch andere übliche hämatologische Tumor-Immunphänotypisierungs-Antikörper, um festzustellen, ob es eine abnormale Expression der Antigene verwandter Untergruppen gibt,
wobei der Lactoferrin-Antikörper, der Lysozym-Antikörper, der CD45-Antikörper und die anderen üblichen hämatologischen Tumor-Immunphänotypisierungs-Antikörper jeweils Metallmarkierungen aufweisen und die Metallmarkierungen der Antikörper unterschiedlich sind.

2. Verfahren nach Anspruch 1, wobei die jeweilige Metall-Markierung ausgewählt ist aus 89Y, 115In, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 157Gd, 158Gd, 159Tb, 160Gd, 161Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 195Pt, 197Au, 198Pt, und 209Bi.

## Revendications

1. Procédé de gating pour l'immunophénotypage hématologique des tumeurs par cytométrie de masse, comprenant les étapes suivantes :
(1) la distinction d'un sous-ensemble de granulocytes matures, d'un sous-ensemble de monocytes et d'autres sous-ensembles cellulaires au moyen d'un anticorps anti-lactoferrine et d'un anticorps anti-lysozyme ;
(2) la distinction des autres sous-ensembles cellulaires au moyen d'un anticorps anti-CD45, y compris un sous-ensemble de cellules primitives et juvéniles ou/et un sous-ensemble de cellules anormales, un sous-ensemble de globules rouges nucléés et un sous-ensemble de lymphocytes ; et
(3) l'analyse de l'expression des antigènes des sous-ensembles au moyen d'autres anticorps d'immunophénotypage de tumeurs hématologiques communes afin de déterminer s'il existe une expression anormale des antigènes des sous-ensembles apparentés,
où l'anticorps de lactoferrine, l'anticorps de lysozyme, l'anticorps de CD45 et les autres anticorps d'immunophénotypage de tumeurs hématologiques communes comportent respectivement des marqueurs métalliques, et les marqueurs métalliques des anticorps sont différents.

2. Procédé selon la revendication 1, dans lequel l'étiquette métallique respective est choisie parmi 89Y, 115In, 139La, 141Pr, 142Nd, 143Nd, 144Nd, 145Nd, 146Nd, 147Sm, 148Nd, 149Sm, 150Nd, 151Eu, 152Sm, 153Eu, 154Sm, 155Gd, 156Gd, 157Gd, 158Gd, 159Tb, 160Gd, 161 Dy, 162Dy, 163Dy, 164Dy, 165Ho, 166Er, 167Er, 168Er, 169Tm, 170Er, 171Yb, 172Yb, 173Yb, 174Yb, 175Lu, 176Yb, 195Pt, 197Au, 198Pt et 209Bi.
